(19) Europäisches Patentamt / European Patent Office / Office européen des brevets

(11) **EP 3 934 538 B1**

(12) **EUROPEAN PATENT SPECIFICATION**

(45) Date of publication and mention
of the grant of the patent:
**03.08.2022 Bulletin 2022/31**

(21) Application number: **20707134.1**

(22) Date of filing: **05.03.2020**

(51) International Patent Classification (IPC):
*A61B 6/02* *(2006.01)*    *A61B 6/10* *(2006.01)*
*A61B 6/00* *(2006.01)*    *A61B 6/03* *(2006.01)*

(52) Cooperative Patent Classification (CPC):
**A61B 6/4441; A61B 6/027; A61B 6/032;**
**A61B 6/501; A61B 6/54;** A61B 6/102

(86) International application number:
**PCT/EP2020/055807**

(87) International publication number:
**WO 2020/178371 (10.09.2020 Gazette 2020/37)**

(54) **APPARATUS FOR DETERMINING A CONTROL PROTOCOL FOR CONTROLLING A C-ARM SYSTEM**

VORRICHTUNG ZUR BESTIMMUNG EINES STEUERUNGSPROTOKOLLS ZUR STEUERUNG EINES C-BOGEN-SYSTEMS

APPAREIL DE DÉTERMINATION D'UN PROTOCOLE DE COMMANDE POUR CONTRÔLER UN SYSTÈME DE BRAS EN C

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.03.2019 EP 19161299**

(43) Date of publication of application:
**12.01.2022 Bulletin 2022/02**

(73) Proprietor: **Koninklijke Philips N.V.**
**5656 AG Eindhoven (NL)**

(72) Inventors:
• **HUMMEL, Erik**
**5656 AE Eindhoven (NL)**
• **VAN NIJNATTEN, Fred Simon Berend**
**5656 AE Eindhoven (NL)**
• **VAN DE HAAR, Peter George**
**5656 AE Eindhoven (NL)**
• **WITHAGEN, Petrus Johannes**
**5656 AE Eindhoven (NL)**
• **VAN ROOIJEN, Joost Adrianus**
**5656 AE Eindhoven (NL)**

(74) Representative: **Philips Intellectual Property & Standards**
**High Tech Campus 52**
**5656 AG Eindhoven (NL)**

(56) References cited:
DE-A1-102012 211 998    US-A1- 2002 168 053
US-A1- 2006 023 830    US-A1- 2012 106 708
US-A1- 2018 365 869

**Description**

FIELD OF THE INVENTION

The invention relates to an apparatus, a system, a method and a computer program for determining a control protocol for controlling a C-arm system.

BACKGROUND OF THE INVENTION

Today, many interventional procedures are guided by the use of medical imaging. Since during a medical procedure it is often not possible to introduce the patient into a closed CT system, often C-arm systems are used for acquiring computed tomography imaging data in applications in which providing a 3D x-ray tomography image is advantageous. In this context, to acquire the tomographic data, a radiation source and a detector provided by the C-arm are moved on a circular traj ectory in a vertical plane around a region of interest of the patient. This can be easily achieved by simply rotating the C-arm around a propeller joint about a propeller angle of more than 180 degrees (°).

Although in many applications this method leads to CT images, in particular cone beam CT images, with an adequate quality, in some applications, especially with respect to the head of the patient, image artifacts might occur due to shadowing effects of high attenuation objects like the teeth of the patient. Moreover, a region of interest can be vertically aligned with anatomical structures being highly sensitive to x-ray radiation such that with the above described method these highly sensitive regions will be subject to high intensity radiation during the imaging of the region of interest.

US 2002/168053 A1 discloses an X-ray device for the acquisition of a set of projection images for the reconstruction of a 3D image data set of an object that is arranged in an examination zone, wherein the X-ray device includes an X-ray source and an X-ray detector for the acquisition of the projection images of the object and a control unit for guiding the X-ray source along a trajectory around the object. The trajectory is situated essentially on a spherical surface in order to acquire the projection images, wherein the X-ray device is constructed in such a manner that the X-ray source can continuously follow the

trajectory in order to acquire the set of projection images and such that not all points of the trajectory are situated in a common plane.

SUMMARY OF THE INVENTION

It is an object of the present invention to provide an apparatus, a system comprising the apparatus, a method and a computer program that allow acquiring computed tomographic images with an improved quality using a C-arm system. Moreover, it is a further object of the present invention that the apparatus, the system, the method and the computer program also allow reducing radiation in radiation-sensitive areas.

In a first aspect of the present invention an apparatus for determining a control protocol for controlling a C-arm system comprising a radiation source is presented, wherein the control protocol comprises a sequence of roll angles and a sequence of propeller angles, wherein the apparatus comprises a) a tilt plane providing unit for providing a tilt plane tilted with respect to a vertical plane, and b) a control protocol determining unit for determining a control protocol by determining the sequence of roll angles and the sequence of propeller angles such that the radiation source follows a trajectory that comprises a circular component in the tilt plane and that allows the C-arm system to acquire projection data for image reconstruction.

Since the control protocol determining unit determines a control protocol for controlling the C-arm system by determining the sequence of roll angles and the sequence of propeller angles such that the radiation source follows a trajectory that comprises a circular component in the tilt plane with respect to a vertical plane and that allows the C-arm system to acquire projection data for image reconstruction, preferably tomographic images, the tilt plane can be chosen such that during the acquisition of projection data for tomographic image reconstruction the region of interest is imaged while highly attenuating objects near the region of interest can be avoided. Moreover, also radiation-sensitive regions near the region of interest can be avoided by choosing the tilt plane accordingly. Therefore, the apparatus allows to provide a control protocol for the C-arm system that allows the acquisition of a CT image with an improved image quality while at the same time allowing to reduce radiation exposure to radiation-sensitive areas.

The apparatus is adapted to determine a control protocol for controlling a C-arm system. A C-arm system refers to an x-ray system comprising a radiation source and a detector provided, for instance, on a C-arm. In some embodiments, a C-arm system comprises two robotic arms on which the radiation source and the detector are provided, wherein the two robotic arms can be operated independently. In other embodiments only the radiation source is moved, whereas the detector has a fixed position. Preferably, the C-arm system is a cone beam C-arm CT system. In general, a C-arm system is configured to rotate the radiation source and/or the detector around at least two axes. The two axes of movement of the C-arm system both lie in a horizontal plane and are perpendicular to each other. One of the two axes is called the propeller axis and the other of the two axes is called the roll axis. Accordingly, a movement of the radiation source can be controlled by controlling the movement of the C-arm around the propeller axis and the roll axis. The apparatus is adapted to determine, for controlling the C-arm system, a control protocol comprising a sequence of roll angles and a sequence of propeller angles defined based on the propeller axis and the roll axis, respectively. The sequence of roll angles and the sequence of propeller angles defines a sequence of particular positions of the radiation source in space, such that the sequence of positions forms a trajectory of the radiation source in space. The trajectory of the radiation source provided on the C-arm of the C-arm system thus refers to a sequence of positions of the radiation source determined by the sequence of roll angles and the sequence of propeller angles provided by the control protocol. Accordingly, the control protocol allows to control the movement of a C-arm system such that the radiation source follows a specific trajectory during the acquisition of projection data for tomographic image reconstruction.

The tilt plane providing unit is adapted to provide a tilt plane tilted with respect to a vertical plane. The tilt plane providing unit can be, for instance, a storage unit storing the tilt plane or the tilt plane providing unit can be connected to a storage unit storing the tilt plane. Moreover, the tilt plane providing unit can be a receiving unit adapted to receive the tilt plane, for instance, through an input unit into which a user can input the tilt plane, wherein the tilt plane providing unit is then adapted to provide the tilt plane.

The tilt plane is tilted with respect to a vertical plane. In particular, a tilt angle can be defined between a normal of the tilt plane and a normal of the vertical plane, defining an amount of tilt of the tilt plane with respect to the vertical plane. The tilt plane is tilted with respect to the vertical plane such that the tilt angle, i.e. the amount of tilt, is always greater than zero and smaller than 90 degrees (°), wherein the tilt angle corresponds to an absolute value. In a preferred embodiment the tilt angle is smaller than 20° and greater than zero. More preferably, if the object to be imaged is a human patient lying on a patient support, the vertical plane refers substantially to an axial plane of the patient. Alternatively, the vertical plane can refer substantially to a sagittal plane of the patient. Moreover, the vertical plane can also be defined with respect to other cross sections through the patient depending on the position of the patient with respect to the C-arm system. Preferably, the tilt plane is defined such that the intersection curve, i.e. the intersection line, of the tilt plane and the vertical plane lies within a horizontal plane. Even more preferably, the intersection curve of the tilt plane and the vertical plane corresponds to the roll axis or the propeller axis of the C-arm system.

The control protocol determining unit is adapted to determine the control protocol. Determining the control protocol comprises determining the sequence of roll angles and the sequence of propeller angles of the C-arm system such that the radiation source follows a trajectory that comprises a circular component in the tilt plane. In particular, the control protocol determining unit is adapted to determine the roll angle depending on the propeller angle for determining the control protocol. Generally, a trajectory of an object in space can be described as the superposition of a plurality of movement components. For example, a linear movement in an arbitrary direction can be described as a superposition of movements along a horizontal and a vertical axis chosen appropriately. The control protocol determining unit is adapted to determine the control protocol for the radiation source such that the trajectory of the radiation source comprises at least a circular component in the tilt plane. A circular component corresponds to the movement of the radiation source along a circular path, i.e. a path that forms a circle or a section of a circle, if the radiation source follows a trajectory only comprising the circular component. The circular component of the trajectory lies in the tilt plane so that the radiation source would not leave the tilt plane when following a trajectory only comprising the circular component. Moreover, the radiation provided by the radiation source is provided along the tilt plane such that at least a part of the provided radiation is provided within the tilt plane and follows a path within the tilt plane to the detector. For example, the control protocol determining unit can be adapted to determine a circular component as defined above by using a sine or cosine function for calculating the roll angle in dependence of the propeller angle, wherein the function can be chosen such that the amplitude of the sine or cosine function corresponds to the tilt angle. Alternative, other methods, i.e. mathematical equations, for determining and calculating a corresponding circular component can be chosen. In a further example, the control protocol determining unit can be adapted for determining the control protocol to firstly determine a desired trajectory of the radiation source in space and then to determine the sequence of roll angles and the sequence of propeller angles such that the radiation source of the C-arm system follows the determined trajectory.

The circular component in the tilt plane of the trajectory of the radiation source allows to acquire projection data from a region of interest without acquiring, for instance, projection data from high attenuation regions near the region of interest, which might lead to artifacts during tomographic image reconstruction of the projection data.

The trajectory defined by the control protocol determined by the control protocol determining unit further allows the C-arm system to acquire projection data for tomographic image reconstruction. In particular, the trajectory allows the acquisition of a full projection data set, for instance, by providing projection data from a plurality of different views over 180° plus fan angle. But, depending on the geometry of the radiation provided by the radiation source, also views provided over less than 180° can be used to build a full set of projection data. To ensure that the trajectory allows to acquires projection data that can be used for tomographic image reconstruction, the control protocol defining the trajectory of the radiation source can be determined also according to the principles for acquiring a Cone Beam CT scan, as disclosed, for instance, in the book "Cone Beam Computed Tomography" by Chris C. Shaw, 1st Edition, CRC press (2014).

In an embodiment, the trajectory further comprises a non-circular component. Preferably, the control protocol determining unit is adapted to determine the control protocol such that the radiation source follows a trajectory comprising a circular component and the non-circular component, i.e. such that the trajectory of the radiation source is a superposition of the circular and the non-circular component. The non-circular component can be chosen by the user such that additional constraints of the movement of the trajectory can be fulfilled. For instance, the non-circular component can be chosen such that the radiation source follows a trajectory that allows to provide projection data outside of the tilt plane at certain positions. In another example, the non-circular component can be chosen such that the radiation source follows a trajectory with differing distances to the object to be imaged, for instance, an elliptical trajectory in the tilt plane. In a preferred embodiment, the non-circular component allows the C-arm system to acquire projection data along a trajectory that fulfills Tuy's condition if the C-arm system is controlled in accordance with the determined control protocol. Generally, a trajectory of a radiation source external to the object satisfies Tuy's condition if any plane through the object is intersected by the trajectory. A more detailed explanation of trajectories fulfilling Tuy's condition can be found in the article "An inversion formula for cone-beam reconstruction" by H. K. Tuy, SIAM J. Appl. Math., volume 43(3), pages 546 to 552, (1983).

In a preferred embodiment, the non-circular component comprises a back-and-forth movement component of the radiation source perpendicular to the tilted plane. A back-and-forth movement component of the radiation source perpendicular to the tilt plane superposed on the circular component can allow the radiation source to follow a trajectory that fulfills Tuy's condition with respect to the region of interest that should be imaged. Preferably, the non-circular component comprises a back-and-forth movement component that moves the radiation source back and forth perpendicular to the tilt plane at least two times during a semicircle movement of the radiation source.

To determine if Tuy's condition is fulfilled by a possible trajectory, for instance, comprising as non-circular component a back-and-forth movement component, a convex hull of the trajectory can be calculated and in a 3D viewer overlaid with a representation of the object to be images. In this case Tuy's condition is fulfilled for a possible trajectory if the object, or a region of interest of the object can be found within the convex hull of the trajectory. If it is desired to determine a control protocol defining a trajectory that fulfills Tuy's condition, the control protocol determining unit can be adapted to adapt the characteristics of the trajectory such that the trajectory fulfills Tuy's condition, wherein the control protocol determining unit can then determine the control protocol such that the radiation source follows the desired trajectory. The characteristics of the trajectory can refer to, for instance, start and end points of the trajectory, maximum and/or minimum values of the roll angle, a position of maxima and/or minima of the trajectory, etc., wherein adapting these characteristics can also refer to adapting the non-circular component of the trajectory, for instance, adapting a back-and-forth movement component such that the characteristics of the trajectory are adapted. Moreover, it is preferred that a starting and ending part of the trajectory is chosen such that the X-ray system can easily follow the dynamics of the trajectory.

In an embodiment, the apparatus further comprises a table position providing unit for providing a position and orientation of a table with respect to the C-arm system, wherein an object that should be imaged by the C-arm system is positioned on the table, wherein the control protocol determining unit is adapted to determine the sequence of roll angles and the sequence of propeller angles further based on the position and orientation of the table. The table position providing unit can be a storage unit storing the table position or can be connected to a storage unit storing the table position. Moreover, the table position providing unit can be a receiving unit receiving the table position and being adapted to then provide the received table position. The table position can, for instance, be received from an input unit into which a user inputs the table position or from a table position determination unit. Such a table position determination unit can, for instance, determine a table position based on measurements, for instance, optical measurements, of the table position. An object that should be imaged by the C-arm system is positioned on the table, wherein the object can be a living being like a human or an animal or can be an inanimate object like a suitcase. Preferably, the object is a patient of which a medical image should be acquired using the C-arm system.

The control protocol determining unit is then adapted to further determine the sequence of roll angles and the sequence of propeller angles based on the position and orientation of the table. For instance, if a longitudinal axis of the table is aligned with a propeller axis of the CT system, the control protocol determining unit is adapted to determine the sequence of roll angles and the sequence of propeller angles such that the sequence of propeller angles provides the circular movement of the radiation source around the table and the sequence of roll angles provides the tilt of the trajectory with respect to the vertical plane. In another example, if the table is oriented such that a longitudinal axis of the table is aligned with a roll axis of the C-arm system, the control protocol determining unit is adapted to determine the sequence of roll angles and the sequence of propeller angles such that the roll angles provide the circular movement of the radiation source around the table and the propeller angles provide the tilt of the trajectory with respect to the vertical plane. Moreover, the positions and orientations of the table can also be arbitrary positions and orientations of the table with respect to the C-arm system and the control protocol determining unit can be adapted to determine the sequence of roll angles and the sequence of propeller angles with respect to these arbitrary positions and orientations of the table such that the radiation source follows a trajectory with a circular component in the provided tilt plane. Preferably, the position and orientation of the table also defines substantially the position and orientation of the object, preferably the patient, with respect to the C-arm system. Therefore, when the control protocol determining unit is adapted to determine the control protocol based on the position and orientation of the table, the control protocol determining unit is also adapted to determine the control protocol with respect to the position and orientation of the object provided on the table. Moreover, if the table position and orientation is provided by the table position providing unit, the tilt plane providing unit can be adapted to provide the tilt plane with respect to the position and orientation of the table. For instance, the vertical plane can be defined with respect to the position and orientation of the table or the object on the table such that also the tilt plane is defined based on the position and orientation of the table and/or the object on the table.

In an embodiment, the control protocol determining unit is adapted to determine the sequence of roll angles and the sequence of propeller angles such that a risk of a collision between a part of the C-arm system and the table and/or the object is minimized during the acquisition of the projection data. For instance, the control protocol determining unit can be adapted to determine the sequence of roll angles and the sequence of propeller angles such that the radiation source or another part of the C-arm system, for instance, of the C-arm, is never in the same position as a part of the table and/or the object when the radiation source follows the predetermined trajectory. The control protocol determining unit can, for instance, be adapted to use the position of the table and/or the object as restricting condition for the determination of the trajectory. Alternatively or additionally, the control protocol determining unit can, for instance, be adapted to determine a first trajectory and to check whether the first determined trajectory comprises a risk of collision between a part of the C-arm system and the table and/or patient. If such a risk of collision is determined the control protocol determining unit can be adapted to determine a new trajectory, for instance, with a different starting position or with a different non-circular component, wherein new trajectories are determined until a trajectory is found for which no risk of collision is determined.

In an embodiment, the control protocol determining unit is adapted to determine the sequence of roll angles and the sequence of propeller angles such that the radiation source passes through a horizontal plane comprising the table and/or the object only once during an acquisition of projection data. If the radiation source passes through a horizontal plane comprising the table and/or the patient only once during an acquisition of the projection data, a danger of collision arises only once during the acquisition of the CT image. Thus, the risk of collision between the C-arm system and the table and/or the object can be reduced.

In an embodiment, the control protocol determining unit is adapted to determine the sequence of roll angles and the sequence of propeller angles such that a roll angle is substantially zero for all propeller angles at which the radiation source passes through a horizontal plane comprising the table and/or the object, if the table is positioned such that a longitudinal axis of the table is parallel to a propeller axis of the C-arm system. In a case in which the table is positioned such that a longitudinal axis of the table is substantially aligned with a propeller axis of the C-arm system, a roll angle of substantially zero degrees for all propeller angles at which the radiation source passes through a horizontal plane comprising the table and/or the patient reduces the risk of collisions. Preferably, the control protocol determining unit is adapted to determine a non-circular component such that the superposed trajectory comprising the circular component and the non-circular component can be achieved with a roll angle being substantially zero for the propeller angles at which the radiation source passes through the horizontal plane comprising the table and/or the patient. In a preferred embodiment, the circular component is combined with the non-circular component such that positive or negative movements in a roll angle direction of one component cancel positive or negative movements in a roll angle direction of another component for propeller angles at which the radiation source passes through the horizontal plane comprising the table and/or the object.

In an embodiment, the control protocol determining unit is adapted to determine the sequence of roll angles and the sequence of propeller angles such that a roll angle is below 20° for propeller angles at which the radiation source is below the table if the table is positioned such that a longitudinal axis of the table is parallel to a propeller axis of the C-arm system. Moreover, the roll angle can also be below 10°. Keeping the roll angle small, for instance, below 20°, for propeller angles at which the radiation source is below the table reduces the risk of collisions between the C-arm and the table substantially and thus increases the safety of the CT image acquisition procedure.

In an embodiment, the control protocol determining unit is adapted to determine the sequence of roll angles and the sequence of propeller angles such that a movement of the radiation source around more than one rotation axis is minimized. Preferably, the control protocol determining unit is adapted to determine the sequence of roll angles and the sequence of propeller angles such that the roll angles are minimized, to minimize an acceleration of the C-arm of the C-arm system. Large roll angles lead to a large acceleration of the C-arm of the C-arm system, wherein such large accelerations can lead to vibrations of the C-arm that result in image artifacts in a reconstruction of the acquired projection data. Therefore, minimizing the roll angle during the acquisition of the projection data allows for the reconstruction of a tomographic image with a higher image quality.

In an embodiment, the control protocol determining unit is adapted to determine the sequence of roll angles and the sequence of propeller angles such that an acceleration in a direction parallel to an anode rotation axis of the radiation source is minimized. In a conventional C-arm system, the anode of a radiation source is rotated around an anode rotation axis to provide the radiation to the detector. If the control protocol is determined such that an acceleration in the direction parallel to the anode rotation axis of the radiation source is minimized, the gyroscopic load on the radiation tube can be minimized. This leads to an increased stability of the C-arm system during the acquisition of the projection data for tomographic reconstruction and therefore to an increased quality of the reconstructed image.

In a further aspect of the present invention a system for controlling a C-arm system is presented comprising a) an apparatus as described above for determining a control protocol for controlling a C-arm system, and b) a control unit for controlling the C-arm system in accordance with the determined control protocol.

In a further aspect of the present invention a method for determining a control protocol for controlling a C-arm system comprising a radiation source is presented, wherein the control protocol comprises a sequence of roll angles and a sequence of propeller angles, wherein the method comprises the steps of a) providing a tilt plane tilted with respect to a vertical plane, and b) determining a control protocol by determining the sequence of roll angles and the sequence of propeller angles such that the radiation source follows a trajectory that comprises a circular component in the tilt plane and that allows the C-arm system to acquire projection data for image reconstruction.

In a further aspect of the present invention a computer program for providing a control protocol for a C-arm system is presented, wherein the computer program comprises program code means for causing the apparatus described above to carry out the steps of the method as described above when the computer program is executed on the system.

It shall be understood that the apparatus of claim 1, the system of claim 12, the method of claim 13, and the computer program of claim 14 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

BRIEF DESCRIPTION OF THE DRAWINGS

These and other aspects of the system and the method according to the invention will be further elucidated and described with reference to the drawing, in which:

Fig. 1 shows schematically and exemplarily an embodiment of a system comprising an apparatus for providing a control protocol for a C-arm system,
Fig. 2 shows a schematic drawing explaining in more detail a principle of the present invention,
Fig. 3 shows an exemplary sequence of roll and propeller angles of one embodiment of the apparatus,
Fig. 4 shows an exemplary sequence of roll angles and propeller angles of a known control protocol and a control protocol according to an embodiment of the present invention, and
Fig. 5 shows a flowchart exemplarily illustrating an embodiment of a method for providing a control protocol for controlling a C-arm system.

DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail.

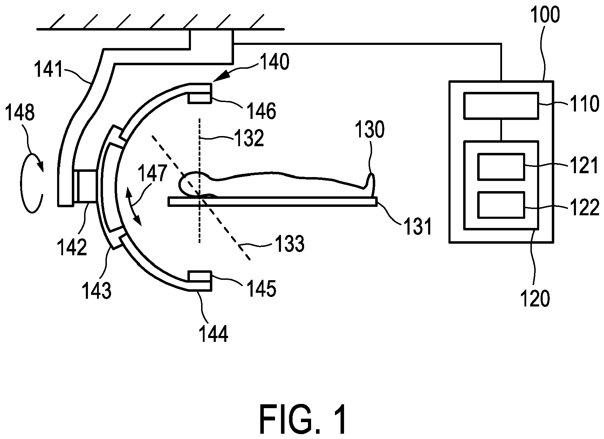

Fig. 1 shows schematically and exemplarily an embodiment of a system for controlling a C-arm system comprising an apparatus for determining a control protocol for controlling the C-arm system. The system 100 comprises a control unit 110 for controlling the movement of a C-arm 144 of the C-arm system 140 in accordance with the control protocol determined by apparatus 120.

The exemplary C-arm system 140 of this embodiment comprises attachment means 141 that can be movably and/or rotatably attached to a ceiling, or a wall or a floor of a room comprising the C-arm system 140. Further, the C-arm system 140 comprises the C-arm 144 that is attached to the attachment means 141 through roll means 143 and propeller means 142. The propeller means 142 allow a rotational movement 148 around a propeller axis of the C-arm 144 defined by the propeller means 142. In a preferred embodiment as shown here, the propeller means 142 provide a propeller axis that lies in a horizontal plane. The roll means 143 allow a roll movement 147 of the C-arm 144 around a roll axis. In the shown preferred embodiment, the roll axis also lies in a horizontal plane and is perpendicular to the propeller axis. The C-arm 144 further comprises a radiation source 145 and a radiation detector 146 for detecting radiation from the radiation source 145 that has passed through an object, like patient 130, lying on table 131.

The control unit 110 is adapted to control the movements of the C-arm 144. In particular, the control unit 110 controls the movements of the C-arm 144 with respect to the propeller movement 148 and the roll movement 147. Moreover, the control unit 110 is adapted to control the C-arm system 140 such that the radiation source 145 follows a sequence of positions determined by specific roll and propeller angles defined in the control protocol provided by the apparatus 120. At each of the positions provided by the control protocol an image of the patient 130 can be acquired. When starting the C-arm system 140 from a non-moving position of the C-arm 144, the control unit 110 can further be adapted to control the movements of the C-arm 144 such that the C-arm 144 is accelerated slowly and, if a desired speed of the C-arm 144 is reached, starts to follow the sequence of positions provided by the control protocol. For instance, the control unit 110 can be adapted to control the C-arm 144 in accordance with a predetermined acceleration trajectory of the radiation source 145 when starting the movement of the C-arm 144, whereas the control unit 110 can then be adapted to control the C-arm 144 such that the acceleration trajectory is connected to the trajectory of the radiation source 145 provided by the control protocol without interruption such that the acceleration trajectory and the trajectory provided by the control protocol result in a continuous movement of the C-arm 144. Preferably, the control unit 110 controls the C-arm system 140 such that the acquisition of the projection data, i.e. of the image of the patient 130, is started after the acceleration trajectory has been followed through.

The apparatus 120 is adapted to determine a control protocol for the C-arm system 140. The apparatus 120 comprises a tilt plane providing unit 121 and a control protocol determining unit 122. The tilt plane providing unit 121 is adapted to provide a tilt plane 133 tilted with respect to a vertical plane 132. For instance, the tilt plane providing unit can be configured to receive a desired tilt plane 133 from a user and provide the received tilt plane 133. Preferably, the tilt plane providing unit receives a desired tilt angle between the tilt plane 133 and the vertical plane 132 together with a desired tilting direction and provides the tilt plane 133 based on the tilt angle and the tilting direction. Alternatively, the tilt angle can be provided together with a positive or negative sign to indicate the direction of the tilting of the tilt plane, wherein the relation between the positive and negative sign and the direction of tilting can be based on a predetermined convention. Moreover, the tilt plane providing unit 121 can be configured to provide a plurality of possible trajectories, a plurality of fixed tilt angles and/or a plurality of fixed tilt planes to a user, wherein the tilt plane providing unit 121 is then adapted to provide the tilt plane based on the choices of the user.

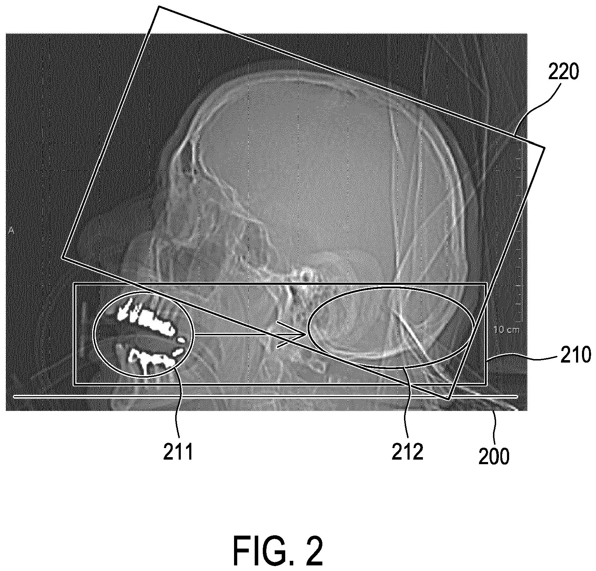

The control protocol determining unit 122 is adapted to determine a control protocol by determining a sequence of roll angles and a sequence of propeller angles such that the radiation source 145 follows a trajectory that comprises a circular component in the tilt plane 133 and that further allows the C-arm system 140 to acquire projection data for tomographic image reconstruction. For instance, if the trajectory of the radiation source 145 comprises only the circular component in the tilt plane, the control protocol determining unit 122 is adapted to determine the roll angles provided by the roll means 143 for each propeller angle provided by the propeller means 142 such that the radiation source 145 lies within tilt plane 133. In the embodiment shown here this corresponds to the radiation source 145 providing radiation within the tilt plane 133 to detector 146. The advantages of such a trajectory are shown in Fig. 2.

In Fig. 2, an image slice 200 of a tomographic image reconstruction of a patient 130 is shown. The tomographic image of patient 130 was acquired in this case through conventional tomographic image acquisition using a C-arm system like the C-arm system 140, wherein no tilt plane 133 was defined and the trajectory of the radiation source 145 comprises a main circular component within a vertical plane 132. This is schematically indicated by the orientation of box 210. In this case, the projection of the teeth of the patient, which can be regarded as highly attenuating objects, shown in region 211, lead to image artifacts that are very prominent, for instance, in region 212 during image reconstruction. In particular, the image artifacts are very prominent in region 212, lying within a part of the image reconstruction that is of interest for a diagnosis of the patient, for instance, in cases of injuries to the brain that are often assessed using tomographic imaging of the head of the patient. Fig. 2 further shows schematically the situation in which the image is acquired by a radiation source 145 following a circular component that lies within the tilt plane 133. In this case, as schematically indicated by box 220, the radiation provided by the radiation source 145 to the detector 146 follows a path that will not lead to image artifacts of the teeth of the patient within the region of interest in the head of the patient, like the brain. Accordingly, providing a tilt plane 133 allows to choose a path for the radiation provided by the radiation source 145 that allows to avoid image artifacts due to, for instance, high attenuating objects in a region of interest.

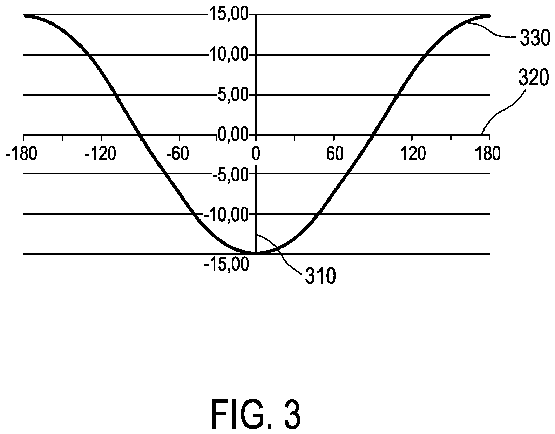

In the following, some exemplary trajectories will be discussed, wherein for these trajectories it is assumed that the table 131 and the object are in a head position, i.e. the longitudinal axes of the table 131 is oriented parallel to the propeller axis such that the propeller axis corresponds substantially to a line through the middle of the table 131. Fig. 3 shows an exemplary representation of a sequence of propeller angles about the propeller axis 320 and roll angles about the propeller axis 310 that defines a traj ectory of the radiation source 145 in the tilt plane 133 when the tilt plane 133 is defined by a tilt angle of 15°. To define a direction of the tilt plane it is further defined for this case that the C-arm should be tilted by -15° when the radiation source 145 is beneath the table 131. In this exemplary case, a radiation source 145 follows a trajectory that only comprises the circular component within the tilt plane 133. To ensure that the trajectory lies within the tilt plane 133 having a tilt angle of 15° tilted in the direction towards the C-arm system 140, the roll angle must be -15° when the propeller angle is 0°, the roll angle must be 0° when the propeller angle is -90° or +90°, and the roll angle must be + 15° when the propeller angle is -180° or + 180°. This is shown by graph 330.

A trajectory as shown in graph 330 can be determined, for instance, by using the following equation:

$$Roll(\lambda) = A\cos(2\pi\, f\lambda + \phi), \tag{1}$$

wherein *Roll* denotes the roll angle, $\lambda$ is in the range of 0 to 1 and is indicative of the propeller angle, *A* corresponds to the provided tilt angle, f is a chosen frequency of the trajectory and $\phi$ corresponds to a chosen phase shift. The parameter $\lambda$ can be determined by a transformation of the range of the propeller angle. For instance, if the range of the propeller angle reaches from 0° to 360°, this range could be linearly transformed to a range of 0 to 1 for $\lambda$. The frequency f refers to a spatial frequency and indicates a number of oscillations of the C-arm around the roll axis during a complete revolution of the C-arm around the propeller axis.

If the trajectory should only comprise a circular component in the tilt plane, for instance, f can be chosen as 1 and $\phi$ can be chosen as 0. In other embodiments, the parameters f and $\phi$ can be used for realizing other requirements of the trajectory, for instance, for introducing a non-circular component by choosing f greater than 1 or for determining a start and end point of the trajectory by choosing $\phi$ accordingly. Further, $\phi$ might also be used to determine where in the trajectory of the radiation source 145 the maximal roll angle is provided with respect to the position of the table 131. Moreover, also further conditions can be introduced in the above equation. For instance, it is often advantageous to provide a symmetric trajectory, to which applies the condition

$$Roll(0) = -Roll(1). \tag{2}$$

This condition results for the above equation (1) in:

$$\phi = \frac{3}{2}\pi - f\pi. \tag{3}$$

Replacing $\phi$ in the first equation with this relation leads to an equation for the roll angle fulfilling the symmetry condition of a symmetric trajectory.

To allow the C-arm system 140 to acquire projection data for tomographic image reconstruction, a full set of views of the patient 130 has to be acquired. This can be achieved, for instance, by acquiring projection data from a plurality of views over a bit more than 180° about the propeller axis. Accordingly, the control protocol determining unit 122 can be adapted to determine any range of a bit more than 180° from the graph 330 shown in Fig. 3 as a sequence of positions of the radiation source 145 and the respective sequence of roll angles and sequence of propeller angles as control protocol. For instance, in one embodiment the control protocol determining unit 122 can determine the sequence of roll angles and the sequence of propeller angles between -100° and +100° propeller angle of graph 330 as part of the control protocol.

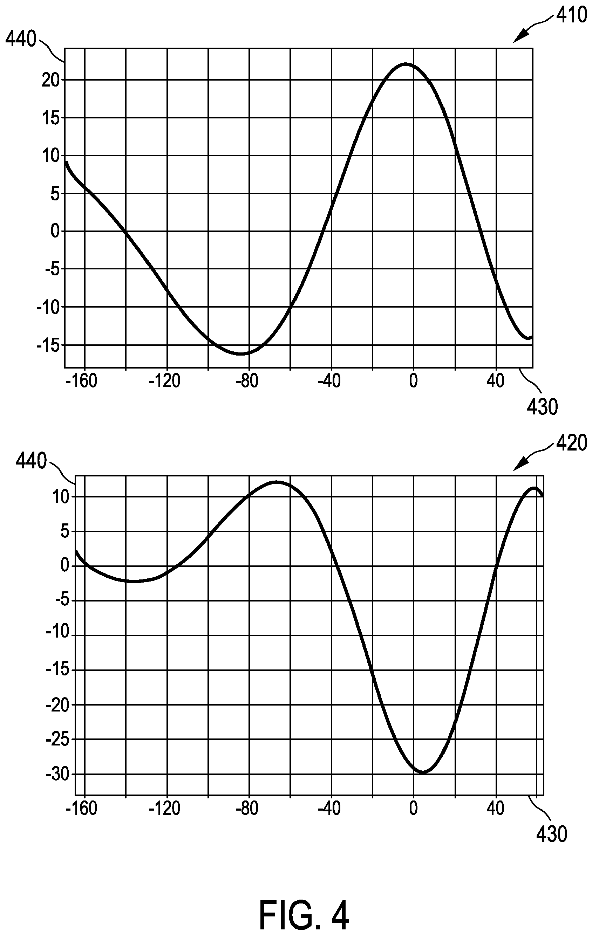

In more advanced embodiments, the trajectory followed by the radiation source 145 can comprise further non-circular components. In these cases, the control protocol determining unit 122 can be adapted, for instance, to determine a non-circular component such that the C-arm system 140 can acquire projection data that fulfills Tuy's condition. In particular, a non-circular component can be introduced that comprises a back-and-forth movement component of the radiation source 145 perpendicular to the tilt plane 133. Moreover, a trajectory can be chirped by introducing further trajectory components, wherein chirping corresponds to providing the further trajectory component such that the resulting component comprises a frequency changing with the propeller angles, as shown, for instance, in graph 420 of Fig. 4. Such a trajectory will be explained in more detail with respect to Fig. 4. Graph 410 shows the chirping, in a commonly known case in which no tilt plane 133 is provided and the radiation source 145 follows a trajectory fulfilling Tuy's condition comprising a circular component lying within vertical plane 132. Axis 440 shows the roll angles and axis 430 shows the propeller angles for such a trajectory when the trajectory is chirped. The chirping leads to a trajectory in graph 410 with a frequency shift from a lower frequency at lower propeller angles to a higher frequency at higher propeller angles.

Graph 420 shows a sequence of roll angles and propeller angles for cases of the present invention comprising a tilt plane 133 with a tilt angle of 15°. To fulfill Tuy's condition the trajectory of the radiation source 145 comprises an additional non-circular component comprising a back-and-forth movement around the roll axis between -17° and +17°. The addition of the back-and-forth movement component is chosen in this case such that for propeller angles between -160° to -90° the back-and-forth movement and the roll movement for the circular component in the tilt plane 133 nearly cancel each other such that only small roll angles for these propeller angles are provided. This leads to a chirping of the trajectory and has the further advantage that collisions with parts of the table 131 or the patient 130 can be avoided.

Accordingly, graph 420 is also an example for a further embodiment of the present invention in which the apparatus 120 further comprises a table position providing unit not shown in Fig. 1 that is adapted to provide a position and orientation of the table 131. In such an embodiment, the control protocol determining unit 122 is adapted to determine the control protocol comprising the sequence of roll angles and the sequence of propeller angles further based on the position and orientation of the table 131. For instance, when the control protocol determining unit 122 is adapted to take the position and orientation of the table 131 into account, the sequence of roll angles and the sequence of propeller angles of the control protocol can be determined such that a risk of collision between parts of the C-arm 144 and parts of the table 131 or the patient 130 can be minimized. For example, if the trajectory of the radiation source 145 only comprises the circular component in the tilt plane 133, the control protocol determining unit 122 can be adapted to choose the sequence of propeller angles in accordance with graph 330 shown in Fig. 3 such that a propeller angle at which the radiation source 145 or the detector 146 lie within a horizontal plane going through the patient 130 or the table 131 is only provided once in the trajectory of the radiation source 145. In the embodiment exemplarily shown here, this would correspond to choosing a sequence of propeller angles that comprises propeller angles around 90° only once, wherein such a sequence could be a sequence of propeller angles between -60° to 120°, respectively.

If an additional non-circular component is introduced into the trajectory of the radiation source 145, for instance, to fulfill Tuy's condition, this additional non-circular component can be chosen to manipulate the trajectory of the radiation source 145 such that the risk of collision between the C-arm 144 and the table 131 or the patient 130 is minimized. An example for such an embodiment is, as already explained above, given in graph 420 of Fig. 4.

A chirped trajectory as shown in graph 420 and discussed above can be determined, for instance, by using the following equation corresponding to an extension of equation (1):

$$Roll(\lambda) = A\sin(2\pi(-f\lambda + \tfrac{1}{2}k\lambda^2 - \tfrac{1}{3}m\lambda^3) + \phi), \qquad (4)$$

wherein in this equation parameters corresponding to the parameters of equation (1) are denoted by the same characters, and $k$ and $m$ are parameters controlling the chirping of the trajectory. The parameters $k$ and $m$ can be used, together with the frequency f, for introducing non-circular components, for instance, for fulfilling Tuy's condition. But, also other conditions can be introduced into the trajectory using the above equation (4). For instance, in some embodiments, it can be advantageous to provide the maximum of the trajectory at $\lambda = 1$. In particular, this condition can be useful for acquiring trajectories that fulfil Tuy's condition, since such a boundary condition makes it more difficult to find a plane through the trajectory of the radiation source 145. If for this case m is chosen to be 0, this condition leads to a phase shift of

$$\phi = \pi(\tfrac{3}{2} + 2f - k). \qquad (5)$$

Replacing $\phi$ in equation (4) with this relation leads to an equation for the roll angle fulfilling the condition that the maximum of this trajectory is always provided at $\lambda = 1$, which, as explained above, makes it easier to find trajectories that fulfil Tuy's condition. Also other conditions for the trajectory can be chosen and introduced into equation (4) in accordance with the given example. It is noted here that the above equations (1) and (4) are only exemplarily for describing a trajectory as defined in the above embodiments and determining corresponding roll angles and propeller angles. Alternatively, these equations could be used in a modified form or other mathematical descriptions, for instance, series expansions like Fourier series, can be used to describe the trajectories and determine the corresponding roll angles and propeller angles under specific conditions.

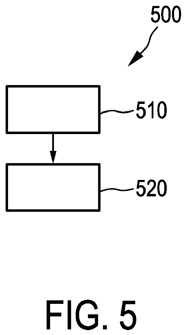

In the following, an embodiment of a method 500 for determining a control protocol for controlling a C-arm system 140 is described with reference to a flowchart shown in Fig. 5. In step 510, a tilt plane 133 tilted with respect to a vertical plane 132 is provided. The tilt plane 133 can be provided, for instance, with reference to an input of a user. In the next step 520, a control protocol is determined by determining a sequence of roll angles and a sequence of propeller angles for the C-arm 144 of the C-arm CT system 140 such that the radiation source 145 follows a trajectory that comprises a circular component in the tilt plane 133 and that allows the C-arm system 140 to acquire projection data for tomographic image reconstruction.

In many applications, Cone Beam CT scans with a cone beam C-arm system are used to visualize soft tissue in three dimensions of the patient, for example, for visualizing a stroke in the head or a tumor in the liver of the patient. Currently, such Cone Beam CTs are made with a circular trajectory of the radiation source, i.e. with a circular trajectory in a vertical plane. With such a trajectory, an exact reconstruction can only be obtained in the plane of rotation. Outside the plane of rotation, i.e. the vertical plane, the Cone Beam CT exhibits artifacts that are stronger than further away from this plane. This is due to not fulfilling Tuy's condition.

An artifact-free tomographic image can be obtained with additional non-circular components of the trajectories. For such a trajectory, the enclosed area, i.e. the convex hull, of the trajectory must enclose the region of interest such that Tuy's condition is fulfilled. Such a trajectory can be realized by a C-arm system, for instance, by a dual-axis scan, i.e. by providing a sequence of propeller angles and roll angles during the acquisition of the projection data. In such a dual-axis scan, i.e. in the acquisition of projection data during which the C-arm of the C-arm CT system moves in accordance with the predetermined control protocol comprising a sequence of propeller angles and a sequence of roll angles, the C-arm moves simultaneously over a primary rotation axis, for instance, the propeller rotation axis, and a secondary rotation axis, for instance, the roll rotation axis. In this case, the system has to be moved over more than 180° around the primary axis in the same way as in a circular scan, while simultaneously moving back and forth over the secondary rotation axis. In an example of neuro-imaging, the C-arm is positioned to scan the head of a patient, the primary rotation axis is realized by the propeller axis, and the secondary rotation axis by the roll axis.

In accordance with the present invention, it is proposed to provide instead of a circular scan in a vertical plane 132 an angulated scan in a tilt plane by tilting the C-arm. For instance, in an angulated head CT scan fewer artifacts of the teeth in the small brain can be expected. Moreover, an x-ray dose on the thyroid, which is the most sensitive organ in this region, can be lowered considerably in this example.

Accordingly, the invention proposes, for instance, an angulated Cone Beam CT scan. A Cone Beam CT scan comprising a circular component in a tilt plane tilted with respect to a vertical plane is beneficial, for instance, for neuro-imaging. For most applications, a slight angulation of the tilt plane of 10° to 25° absolute angle is expected to be sufficient. The angulation, i.e. the movement in the tilt plane, can be provided by the roll movement, for instance, in neuro-imaging in which a table is in a head position, i.e. the table is positioned such that the longitudinal axis of the table is parallel to the propeller axis. In such a case, since the roll sleeve, i.e. roll means, is physically attached to the propeller means and the propeller means moves the C-arm over more than 180° during a scan, the roll angle should not be held constant to realize the movement in the tilt plane. Instead, the roll angle should be adapted continuously to provide the movement in the tilt plane. In addition to a circular component in the tilt plane, the trajectory can also comprise further components, like the movement components used to fulfill Tuy's condition.

For a pure circular scan, i.e. a scan only comprising the circular component in the tilt plane, the angulation, i.e. tilting, causes a positive roll angle below the table in head scanning cases, i.e. at propeller positions below -90° and above +90°. In such an application, the angulation can cause collisions with the table and/or patient. This risk is even increased for obese patients or when patients are positioned off-center. In one embodiment of the invention, it is proposed to provide additional measures when determining the trajectory of the radiation source to avoid or reduce the risk of collisions.

For instance, important aspects of the trajectory that can be taken into account by the control protocol determining unit when determining the sequence of roll angles and the sequence of propeller angles can be a completeness with respect to Tuy's condition, a minimal usage of different rotation axes to increase stability and reproducibility, moderate or minimal accelerations of the radiation source, i.e. x-ray tube, parallel to an axis of anode rotation to minimize gyroscopic load on the tube, minimization of the image acquisition time to avoid motion artifacts and achieve higher spatial resolution by reducing an angular range in a direction of a propeller rotation, or increasing rotation and acceleration speeds, and/or minimization of the risk of collisions.

In particular, in the proposed embodiments, the control protocol determining unit can be adapted to minimize the risk of collision, for instance, by choosing a start position with respect to the position of the table or patient. For instance, a symmetric scan, from -100° to +100° propeller angle, yields two passages of the table and the patient's shoulders in a head scan, whereas, if an open trajectory is chosen, i.e. a trajectory from -80° to +120° of a propeller angle, only one passage of the table and the patient's shoulders in a head scan is provided and thus enough space is given to position the patient such that a collision can be avoided. Moreover, if a non-circular component, for instance, a back-and-forth movement to fulfill Tuy's condition, is added to a circular component in the tilt plane, this non-circular component can be defined such that the roll angle of the overall trajectory of the radiation source is substantially 0° when the C-arm passes the table and/or shoulder of the patient to avoid a collision. For a conventional head scan, this condition applies at approximately -90° or +90°. Further, if a non-circular component is provided, for instance, a back-and-forth movement, to fulfill Tuy's condition, the non-circular component can be chosen such that the overall roll angle of the trajectory of the radiation source is negative below the table. Below the table, a negative roll angle moves the C-arm in a conventional head scan into the free space, whereas a positive angle moves it towards the table and/or patient. For instance, the non-circular component can be chosen such that the negative roll angle of the non-circular component below the table cancels out or reduces the positive roll angle that might be necessary for providing the circular component in the tilt plane. Accordingly, a risk of a collision can be avoided or reduced.

It is further proposed that for specific applications a relaxation of the above described criteria for determining the control protocol may be acceptable in favor of other proposals, for instance, an aspect of providing moderate/minimal accelerations of the x-ray tube parallel to the axis of the anode rotation can be relaxed by allowing, for instance, a chirping of the sinusoidal frequency but fulfilling all other constraints and providing only one lateral crossing. Further, when minimizing the propeller angle to minimize the scan time, Tuy's condition might be violated. But, in moderate cases this can be acceptable, wherein the voxel showing the resulting artifacts can be masked in the reconstructed volume and not shown to the user. Further, depending on the size of the patient, a trajectory may be selected with a lower angulation, i.e. tilting of the tilt plane. For instance, in some applications in which the trajectory comprises a propeller angle of 60° and a roll angle of about 10° during a head scan with a very obese patient, this may cause a collision. In such cases, the tilting of the tilt plane can be reduced to prevent such a collision.

Such trajectories can be realized using a classical C-arm system or a C-arm system which allows a separate motion of the source and the detector using two robotic arms. The latter would also allow a variation of the source detector distance during the acquisition. The trajectories may also be carried out twice with an off-center detector in two different shifted positions in order to achieve a larger field of view.

Although in the above embodiments the C-arm system was a conventional C-arm system, in other embodiments the C-arm system can also be a C-arm system in which the detector and the source can be moved independently from each other using two robotic arms. In such cases, the propeller and roll angles can be defined in accordance with a conventional C-arm system, for instance, with respect to a line of sight between the radiation source and the detector.

Although in the above embodiments the table was provided in a head position, i.e. in a typical position for providing a tomographic image of the head of a patient, in other embodiments the table can be positioned and oriented in a different way. For instance, the table can be oriented perpendicular to the orientation shown in the example above. In such a case, the control protocol determining unit is adapted to determine the sequence of roll angles and the sequence of propeller angles such that the propeller angle provides the tilting of the C-arm necessary for moving the radiation source in a respective tilted plane and the roll angle provides the movement necessary for acquiring projection data for tomographic image reconstruction. Moreover, in other embodiments the table can be oriented and positioned arbitrarily with respect to the C-arm system and the control protocol determining unit is adapted to take the position and orientation of the table into account such that a corresponding trajectory of the radiation source is provided.

Although in the above embodiments the object was a patient, in other embodiments the object can be an animal or even an inanimate object, for instance, a suitcase. Thus, although in the above embodiments the invention was described in the context of a medical application, the invention can also be applied in other contexts, for instance, in a context of a border control.

Although in the above embodiments the region of interest imaged was a head of a patient, in other embodiments other regions of interest like a heart, a liver or any other organ of a patient can be imaged. The principles described with respect to a head of the patient can be applied accordingly to these other regions of interest.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the providing of the tilt plane or the determination of the control protocol, performed by one or several units or devices can be performed by any other number of units or devices. For instance, these procedures can be carried out by a single device. These procedures and/or the control of the apparatus for determining a control protocol can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the computer program may cause a processor or a controller to implement the control method via in accordance with the control protocol. The processor or controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform at the required functions. Various storage media may be fixed within a processor or controller or may be transportable, such that the one or more programs stored thereon can be loaded into a processor or controller.

Any reference signs in the claims should not be construed as limiting the scope.

The invention relates to an apparatus for determining a control protocol for controlling a C-arm system comprising a radiation source. The control protocol comprises a sequence of roll angles and a sequence of propeller angles. The apparatus comprises a tilt plane providing unit for providing a tilt plane tilted with respect to a vertical plane, and a control protocol determining unit for determining a control protocol by determining the sequence of roll angles and the sequence of propeller angles such that the radiation source follows a trajectory that comprises a circular component in the tilt plane and that allows the C-arm system to acquire projection data for tomographic image reconstruction. The apparatus allows to acquire computed tomographic images with an improved quality and to reduce radiation in radiation-sensitive areas.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. An apparatus for determining a control protocol for controlling a C-arm system (140) comprising a radiation source (145), wherein the control protocol comprises a sequence of roll angles and a sequence of propeller angles, wherein the apparatus (120) comprises:

   a tilt plane providing unit (121) for providing a tilt plane (133) tilted with respect to a vertical plane (132), and
   a control protocol determining unit (122) for determining a control protocol by determining the sequence of roll angles and the sequence of propeller angles such that the radiation source (145) follows a trajectory that comprises a circular component in the tilt plane (133) and that allows the C-arm system (140) to acquire projection data for image reconstruction.

2. The apparatus according to claim 1, wherein the trajectory further comprises a non-circular component.

3. The apparatus according to claim 2, wherein the non-circular component allows the C-arm system (140) to acquire projection data along a trajectory that fulfills Tuy's condition if the C-arm system (140) is controlled in accordance with the determined control protocol.

4. The apparatus according to any of claims 2 and 3, wherein the non-circular component comprises a back-and-forth movement component of the radiation source (145) perpendicular to the tilted plane.

5. The apparatus according to any of the previous claims, wherein the apparatus (120) further comprises a table position providing unit for providing a position and orientation of a table (131) with respect to the C-arm system (140), wherein an object (130) that should be imaged by the C-arm system (140) is positioned on the table (131), wherein the control protocol determining unit (122) is adapted to determine the sequence of roll angles and the sequence of propeller angles further based on the position and orientation of the table (131).

6. The apparatus according to claim 5, wherein the control protocol determining unit (122) is adapted to determine the sequence of roll angles and the sequence of propeller angles such that a risk of a collision between a part of the C-arm system (140) and the table (131) and/or the object (130) is minimized during the acquisition of the projection data.

**7.** The apparatus according to any of claims 5 and 6, wherein the control protocol determining unit (122) is adapted to determine the sequence of roll angles and the sequence of propeller angles such that the radiation source (145) passes through a horizontal plane comprising the table (131) and/or the object (130) only once during an acquisition of projection data.

**8.** The apparatus according to any of claims 5 to 7, wherein the control protocol determining unit (122) is adapted to determine the sequence of roll angles and the sequence of propeller angles such that a roll angle is substantially zero for all propeller angles at which the radiation source (145) passes through a horizontal plane comprising the table (131) and/or the object (130) if the table (131) is positioned such that a longitudinal axis of the table (131) is parallel to a propeller axis of the C-arm system (140).

**9.** The apparatus according to any of the claims 5 to 8, wherein the control protocol determining unit (122) is adapted to determine the sequence of roll angles and the sequence of propeller angles such that a roll angle is below 20° for propeller angles at which the radiation source (145) is above the table (131) if the table (131) is positioned such that a longitudinal axis of the table (131) is parallel to a propeller axis of the C-arm system (140).

**10.** The apparatus according to any of the proceeding claims, wherein the control protocol determining unit (122) is adapted to determine the sequence of roll angles and the sequence of propeller angles such that a movement of the radiation source (145) around more than one rotation axis is minimized.

**11.** The apparatus according to any of the proceeding claims, wherein the control protocol determining unit (122) is adapted to determine the sequence of roll angles and the sequence of propeller angles such that an acceleration in a direction parallel to an anode rotation axis of the radiation source (145) is minimized.

**12.** A system for controlling a C-arm system (140) comprising:

an apparatus (120) according to claim 1 for determining a control protocol for controlling a C-arm system (140), and
a control unit (110) for controlling the movement of a C-arm (144) of the C-arm system (140) in accordance with the determined control protocol.

**13.** A method for determining a control protocol for controlling a C-arm system (140) comprising a radiation source (145), wherein the control protocol comprises a sequence of roll angles and a sequence of propeller angles, wherein the method (500) comprises the steps of:

providing (510) a tilt plane (133) tilted with respect to a vertical plane (132), and
determining (520) a control protocol by determining the sequence of roll angles and the sequence of propeller angles such that the radiation source (145) follows a trajectory that comprises a circular component in the tilt plane (133) and that allows the C-arm system (140) to acquire projection data for image reconstruction.

**14.** A computer program for providing a control protocol for a C-arm system (140), wherein the computer program comprises program code means for causing the apparatus of claim 1 to carry out the steps of the method as defined in claim 13 when the computer program is executed on the apparatus.

**Patentansprüche**

**1.** Vorrichtung zum Bestimmen eines Steuerprotokolls zum Steuern eines C-Arm-Systems (140), umfassend eine Strahlungsquelle (145), wobei das Steuerprotokoll eine Sequenz von Rollwinkeln und eine Sequenz von Propellerwinkeln umfasst, wobei die Vorrichtung (120) Folgendes umfasst:

eine Neigungsebenen-Bereitstellungseinheit (121) zum Bereitstellen einer Neigungsebene (133), die in Bezug auf eine vertikale Ebene (132) geneigt ist, und
eine Steuerprotokoll-Bestimmungseinheit (122) zum Bestimmen eines Steuerprotokolls durch Bestimmen der Sequenz von Rollwinkeln und der Sequenz von Propellerwinkeln, sodass die Strahlungsquelle (145) einer Trajektorie folgt, die eine kreisförmige Komponente in der Neigungsebene (133) umfasst und die es dem C-Arm-System (140) ermöglicht, Projektionsdaten zur Bildrekonstruktion zu erfassen.

**2.** Vorrichtung nach Anspruch 1, wobei die Trajektorie ferner eine nicht kreisförmige Komponente umfasst.

**3.** Vorrichtung nach Anspruch 2, wobei die nicht kreisförmige Komponente es dem C-Arm-System (140) ermöglicht, Projektionsdaten entlang einer Trajektorie zu erfassen, die eine Tuy-Bedingung erfüllt, wenn das C-Arm-System (140) gemäß dem bestimmten Steuerprotokoll gesteuert wird.

**4.** Vorrichtung nach einem der Ansprüche 2 und 3, wobei die nicht kreisförmige Komponente eine Hin- und Her-Bewegungskomponente der Strahlungsquelle (145) senkrecht zu der geneigten Ebene umfasst.

**5.** Vorrichtung nach einem der vorherigen Ansprüche, wobei die Vorrichtung (120) ferner eine Tischposition-Bereitstellungseinheit zum Bereitstellen einer Position und Ausrichtung eines Tischs (131) in Bezug auf das C-Arm-System (140) umfasst, wobei ein Objekt (130), das durch das C-Arm-System (140) abgebildet werden soll, auf dem Tisch (131) positioniert ist, wobei die Steuerprotokoll-Bestimmungseinheit (122) angepasst ist, um die Sequenz von Rollwinkeln und die Sequenz von Propellerwinkeln ferner basierend auf der Position und Ausrichtung des Tischs (131) zu bestimmen.

**6.** Vorrichtung nach Anspruch 5, wobei die Steuerprotokoll-Bestimmungseinheit (122) angepasst ist, die Sequenz von Rollwinkeln und die Sequenz von Propellerwinkeln zu bestimmen, sodass ein Risiko einer Kollision zwischen einem Teil des C-Arm-Systems (140) und dem Tisch (131) und/oder dem Objekt (130) während der Erfassung der Projektionsdaten minimiert ist.

**7.** Vorrichtung nach einem der Ansprüche 5 und 6, wobei die Steuerprotokoll-Bestimmungseinheit (122) angepasst ist, um die Sequenz von Rollwinkeln und die Sequenz von Propellerwinkeln zu bestimmen, sodass die Strahlungsquelle (145) durch eine horizontale Ebene umfassend den Tisch (131) und/oder das Objekt (130) nur einmal während einer Erfassung von Projektionsdaten verläuft.

**8.** Vorrichtung nach einem der Ansprüche 5 bis 7, wobei die Steuerprotokoll-Bestimmungseinheit (122) angepasst ist, um die Sequenz von Rollwinkeln und die Sequenz von Propellerwinkeln zu bestimmen, sodass ein Rollwinkel für alle Propellerwinkel, bei denen die Strahlungsquelle (145) durch eine horizontale Ebene verläuft, umfassend den Tisch (131) und/oder das Objekt (130), im Wesentlichen Null ist, wenn der Tisch (131) positioniert ist, sodass eine Längsachse des Tischs (131) parallel zu einer Propellerachse des C-Arm-Systems (140) ist.

**9.** Vorrichtung nach einem der Ansprüche 5 bis 8, wobei die Steuerprotokoll-Bestimmungseinheit (122) angepasst ist, um die Sequenz von Rollwinkeln und die Sequenz von Propellerwinkeln zu bestimmen, sodass ein Rollwinkel für Propellerwinkel unter 20° ist, bei denen die Strahlungsquelle (145) oberhalb des Tischs (131) ist, wenn der Tisch (131) positioniert ist, sodass eine Längsachse des Tischs (131) parallel zu einer Propellerachse des C-Arm-Systems (140) ist.

**10.** Vorrichtung nach einem der vorherigen Ansprüche, wobei die Steuerprotokoll-Bestimmungseinheit (122) angepasst ist, um die Sequenz der Rollwinkel und die Sequenz der Propellerwinkel zu bestimmen, sodass eine Bewegung der Strahlungsquelle (145) um mehr als eine Drehachse minimiert wird.

**11.** Vorrichtung nach einem der vorherigen Ansprüche, wobei die Steuerprotokoll-Bestimmungseinheit (122) angepasst ist, um die Sequenz von Rollwinkeln und die Sequenz von Propellerwinkeln zu bestimmen, sodass eine Beschleunigung in eine Richtung parallel zu einer Anodendrehachse der Strahlungsquelle (145) minimiert wird.

**12.** System zum Steuern eines C-Arm-Systems (140), umfassend:

eine Vorrichtung (120) nach Anspruch 1 zum Bestimmen eines Steuerprotokolls zum Steuern eines C-Arm-Systems (140), und
eine Steuereinheit (110) zum Steuern der Bewegung eines C-Arms (144) des C-Arm-Systems (140) in Übereinstimmung mit dem bestimmten Steuerprotokoll.

**13.** Verfahren zum Bestimmen eines Steuerprotokolls zum Steuern eines C-Arm-Systems (140), umfassend eine Strahlungsquelle (145), wobei das Steuerprotokoll eine Sequenz von Rollwinkeln und eine Sequenz von Propellerwinkeln umfasst, wobei das Verfahren (500) die folgenden Schritte umfasst:

Bereitstellen (510) einer Neigungsebene (133), die in Bezug auf eine vertikale Ebene (132) geneigt ist, und

Bestimmen (520) eines Steuerprotokolls durch Bestimmen der Sequenz von Rollwinkeln und der Sequenz von Propellerwinkeln, sodass die Strahlungsquelle (145) einer Trajektorie folgt, die eine kreisförmige Komponente in der Neigungsebene (133) umfasst und die es dem C-Arm-System (140) ermöglicht, Projektionsdaten zur Bildrekonstruktion zu erfassen.

14. Computerprogramm zum Bereitstellen eines Steuerprotokolls für ein C-Arm-System (140), wobei das Computerprogramm Programmcode-Einrichtungen umfasst, um die Vorrichtung nach Anspruch 1 zu veranlassen, die Schritte des in Anspruch 13 definierten Verfahrens auszuführen, wenn das Computerprogramm auf der Vorrichtung ausgeführt wird.

**Revendications**

1. Appareil pour déterminer un protocole de commande pour commander un système de bras en C (140) comprenant une source de rayonnement (145), dans lequel le protocole de commande comprend une séquence d'angles de roulis et une séquence d'angles d'hélice, dans lequel l'appareil (120) comprend :
une unité de fourniture de plan d'inclinaison (121) pour fournir un plan d'inclinaison (133) incliné par rapport à un plan vertical (132), et une unité de détermination de protocole de commande (122) pour déterminer un protocole de commande en déterminant la séquence d'angles de roulis et la séquence d'angles d'hélice de sorte que la source de rayonnement (145) suit une trajectoire qui comprend un composant circulaire dans le plan d'inclinaison (133) et qui permet au système de bras en C (140) d'acquérir des données de projection pour la reconstruction d'image.

2. Appareil selon la revendication 1, dans lequel la trajectoire comprend en outre un composant non circulaire.

3. Appareil selon la revendication 2, dans lequel le composant non circulaire permet au système de bras en C (140) d'acquérir des données de projection le long d'une trajectoire qui remplit la condition de Tuy si le système de bras en C (140) est commandé conformément au protocole de commande déterminé.

4. Appareil selon l'une quelconque des revendications 2 et 3, dans lequel le composant non circulaire comprend un composant de mouvement de vaet-vient de la source de rayonnement (145) perpendiculaire au plan incliné.

5. Appareil selon l'une quelconque des revendications précédentes, dans lequel l'appareil (120) comprend en outre une unité de fourniture de position de table pour fournir une position et une orientation d'une table (131) par rapport au système de bras en C (140), dans lequel un objet (130) qui doit être imagé par le système de bras en C (140) est positionné sur la table (131), dans lequel l'unité de détermination de protocole de commande (122) est adaptée pour déterminer la séquence d'angles de roulis et la séquence d'angles d'hélice sur la base en outre de la position et de l'orientation de la table (131).

6. Appareil selon la revendication 5, dans lequel l'unité de détermination de protocole de commande (122) est adaptée pour déterminer la séquence d'angles de roulis et la séquence d'angles d'hélice de sorte qu'un risque de collision entre une partie du système de bras en C (140) et la table (131) et/ou l'objet (130) est minimisé pendant l'acquisition des données de projection.

7. Appareil selon l'une quelconque des revendications 5 et 6, dans lequel l'unité de détermination de protocole de commande (122) est adaptée pour déterminer la séquence d'angles de roulis et la séquence d'angles d'hélice de sorte que la source de rayonnement (145) passe au travers d'un plan horizontal comprenant la table (131) et/ou l'objet (130) une seule fois pendant une acquisition de données de projection.

8. Appareil selon l'une quelconque des revendications 5 à 7, dans lequel l'unité de détermination de protocole de commande (122) est adaptée pour déterminer la séquence d'angles de roulis et la séquence d'angles d'hélice de sorte qu'un angle de roulis est sensiblement de zéro pour tous les angles d'hélice auxquels la source de rayonnement (145) passe au travers d'un plan horizontal comprenant la table (131) et/ou l'objet (130) si la table (131) est positionnée de sorte qu'un axe longitudinal de la table (131) est parallèle à un axe d'hélice du système de bras en C (140).

9. Appareil selon l'une quelconque des revendications 5 à 8, dans lequel l'unité de détermination de protocole de commande (122) est adaptée pour déterminer la séquence d'angles de roulis et la séquence d'angles d'hélice de sorte qu'un angle de roulis est inférieur à 20° pour des angles d'hélice auxquels la source de rayonnement (145) est au-dessus de la table (131) si la table (131) est positionnée de sorte qu'un axe longitudinal de la table (131) est parallèle à un axe d'hélice du système de bras en C (140).

10. Appareil selon l'une quelconque des revendications précédentes, dans lequel l'unité de détermination de protocole de commande (122) est adaptée pour déterminer la séquence d'angles de roulis et la séquence d'angles d'hélice de sorte qu'un mouvement de la source de rayonnement (145) autour de plus d'un axe de rotation est minimisé.

11. Appareil selon l'une quelconque des revendications précédentes, dans lequel l'unité de détermination de protocole de commande (122) est adaptée pour déterminer la séquence d'angles de roulis et la séquence d'angles d'hélice de sorte qu'une accélération dans une direction parallèle à un axe de rotation d'anode de la source de rayonnement (145) est minimisée.

12. Système pour commander un système de bras en C (140) comprenant: un appareil (120) selon la revendication 1 pour déterminer un protocole de commande pour commander un système de bras en C (140), et une unité de commande (110) pour commander le mouvement d'un bras en C (144) du système de bras en C (140) conformément au protocole de commande déterminé.

13. Procédé pour déterminer un protocole de commande pour commander un système de bras en C (140) comprenant une source de rayonnement (145), dans lequel le protocole de commande comprend une séquence d'angles de roulis et une séquence d'angles d'hélice, dans lequel le procédé (500) comprend les étapes consistant à:

fournir (510) un plan d'inclinaison (133) incliné par rapport à un plan vertical (132), et
déterminer (520) un protocole de commande en déterminant la séquence d'angles de roulis et la séquence d'angles d'hélice de sorte que la source de rayonnement (145) suit une trajectoire qui comprend un composant circulaire dans le plan d'inclinaison (133) et qui permet au système de bras en C (140) d'acquérir des données de projection pour la reconstruction d'image.

14. Programme informatique pour fournir un protocole de commande pour un système de bras en C (140), dans lequel le programme informatique comprend des moyens de code de programme pour amener l'appareil de la revendication 1 à exécuter les étapes du procédé tel que défini dans la revendication 13 lorsque le programme informatique est exécuté sur l'appareil.

EP 3 934 538 B1

FIG. 1

17

FIG. 2

**FIG. 3**

FIG. 4

500

510

520

# FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2002168053 A1

**Non-patent literature cited in the description**

- **CHRIS C. SHAW.** Cone Beam Computed Tomography. CRC press, 2014

- **H. K. TUY.** An inversion formula for cone-beam reconstruction. *SIAM J. Appl. Math.,* 1983, vol. 43 (3), 546-552